Europäisches Patentamt

European Patent Office

Office européen des brevets

(11) Veröffentlichungsnummer: **0 273 218**
**A1**

(12) # EUROPÄISCHE PATENTANMELDUNG

(21) Anmeldenummer: 87117726.7

(22) Anmeldetag: 01.12.87

(51) Int. Cl.⁴: **A61K 7/00**

(30) Priorität: 24.12.86 DE 3644473

(43) Veröffentlichungstag der Anmeldung:
06.07.88 Patentblatt 88/27

(84) Benannte Vertragsstaaten:
**AT BE CH DE ES FR GB GR IT LI NL SE**

(71) Anmelder: **Wella Aktiengesellschaft**
**Berliner Allee 65**
**D-6100 Darmstadt(DE)**

(72) Erfinder: **Hölzel, Hans**
**Jahnstrasse 9**
**D-6101 Fränkisch-Crumbach(DE)**
Erfinder: **Konrad, Eugen**
**Mecklenburger Strasse 101**
**D-6100 Darmstadt(DE)**
Erfinder: **Mager, Herbert, Dr.**
**Beaumont 5**
**CH-1700 Fribourg(CH)**
Erfinder: **Schwarz, Horst**
**Fliederweg 22 D**
**D-6104 Seeheim(DE)**

(54) Konservierte Haar- und Körperreinigungsmittel sowie Verwendung einer Konservierungsstoff-Kombination.

(57) Haar-und Körperreinigungsmittel, worin eine Kombination aus
(A) einem physiologisch verträglichen Salz der Ameisensäure,
(B) Salicylsäure oder deren physiologisch verträglichem Salz und
(C) einer physiologisch unbedenklichen anorganischen oder aliphatischen organischen Säure enthalten ist sowie die Verwendung dieser Kombination zur Konservierung solcher Mittel. Die neuen Haar-und Körperreinigungsmittel sind sowohl sehr gut konserviert als auch sehr gut verträglich.

EP 0 273 218 A1

## Konservierte Haar-und Körperreinigungsmittel sowie Verwendung einer Konservierungsstoff-Kombination

Haar-und Körperreinigungsmittel enthalten in der Regel einen Konservierungsstoff, welcher die Mittel vor dem Befall durch Mikroorganismen wirksam schützen soll.

Für den Einsatz in Haar-und Körperreinigungsmitteln werden an einen Konservierungsstoff gegensätzliche Anforderungen gestellt. So sollte er einerseits in physiologischer und dermatologischer Hinsicht gut verträglich sein und andererseits über eine gute keimhemmende oder sogar keimtötende Wirkung verfügen. Beide Anforderungen sind meist nur schwer miteinander vereinbar.

Als übliche in Haar-und Körperreinigungsmitteln verwendete Konservierungsstoffe seien beispielsweise Formaldehyd, 5-Brom-5-nitro-1,3-dioxan, p-Hydroxybenzoesäureester, 2,4,4'-Trichlor-2'-hydroxy-diphenylether, 5-Chlor-2-methyl-3-isothiazolon, 2-Methyl-3-isothiazolon und 2-Brom-2-nitropropan-1,3-diol genannt.

In letzter Zeit sind einige Konservierungsstoffe, wie beispielsweise Formaldehyd und 2-Methyl-3-isothiazolon, in den Verdacht geraten, nicht genügend verträglich zu sein. Von konservierend wirkenden Aldehyden und Phenolen ist bekannt, daß diese mit Proteinen reagieren oder mit diesen in denaturierender Weise in Wechselwirkung treten. Bei Aldehyden besteht zudem die Gefahr der Sensibilisierung.

In der Literatur werden Ameisensäure und Salicylsäure bereits als Konservierungsstoffe beschrieben. Allerdings fanden sie bislang in Haar-und Körperreinigungsmitteln keine Verwendung. Die Salicylsäure zeigt in niedriger Konzentration alleine keine ausreichende konservierende Wirkung, während in höheren Konzentrationen eine unerwünschte keratolytische Wirkung auftritt. Die Ameisensäure ist eine unangenehm stechend riechende Säure mit stark korrosiver Wirkung. Ein weiterer Grund, warum Ameisensäure nicht als Konservierungsstoff in Haar-und Körperreinigungsmitteln eingesetzt wird, ist auf deren niedrigen pH-Wert zurückzuführen, bei welchem bereits eine Hydrolyse der üblicherweise in Haar-und Körperpflegemitteln als Tenside eingesetzten Alkylethersulfate auftritt.

Es bestand daher die Aufgabe, ein Haar-und Körperreinigungsmittel mit einem Gehalt an einer Konservierungsstoff-Kombination zur Verfügung zu stellen, welche eine gute konservierende Wirkung besitzt und eine bessere Verträglichkeit aufweist als übliche in derartigen Mitteln verwendete Konservierungsstoffe.

Hierzu wurde gefunden, daß ein wasserhaltiges Haar-und Körperreinigungsmittel mit einem Gehalt an 3 bis 50 Gewichtsprozent eines Tensids, dadurch gekennzeichnet, daß es eine Kombination aus

(A) einem physiologisch verträglichen Salz der Ameisensäure,

(B) Salicylsäure oder deren physiologisch verträglichem Salz und

(C) einer physiologisch unbedenklichen anorganischen oder aliphatischen organischen Säure enthält, die gestellte Aufgabe in hervorragender Weise löst.

Das erfindungsgemäße Mittel wird durch die vorstehend beschriebene Kombination von Konservierungsstoffen sehr gut konserviert und weist eine sehr gute Verträglichkeit auf.

Die Güte der Konservierung der vorstehend beschriebenen Haar-und Körperreinigungsmittel wurde durch den in der Literatur The United States Pharmacopeia, 21. Auflage, (1985), Seite 1151 beschriebenen Konservierungsbelastungstest festgestellt. Hierbei wurden die Haarbehandlungsmittel mit den Mikroorganismen Candida albicans (ATCC Nr. 10231), Aspergillus niger (ATCC Nr. 16404). Escherichia coli (ATCC Nr. 8739), Pseudomonas aeruginosa (ATCC Nr. 9027) und Staphylococcus aureus (ATCC Nr. 6538) verunreinigt und die Entwicklung der Keimzahl über einen Zeitraum von 28 Tagen überwacht.

Während die Verwendung von Natriumformiat oder Salicylsäure jeweils allein, insbesondere bei Befall durch Pilze, wie zum Beispiel Aspergillus niger, nicht zu einer zufriedenstellenden Konservierung der Haar-und Körperreinigungsmittel führt, werden die Mittel durch die Verwendung der vorstehend beschriebene Kombination aus den Konservierungsstoffen (A), (B) und (C) auch gegen Pilze sehr gut konserviert.

Als geeignete physiologisch verträgliche Salze der Ameisensäure (A) seien beispielsweise das Natriumformiat, das Ammoniumformiat, das Kaliumformiat und das Magnesiumformiat genannt, wobei das Natriumformiat bevorzugt ist.

In dem Haar-und Körperreinigungsmittel ist die Komponente (A) in einer Menge von etwa 0,01 bis 2 Gewichts prozent, vorzugsweise 0,1 bis 1,0 Gewichtsprozent, enthalten.

Die Komponente (B) ist in einer Menge von etwa 0,05 bis 2 Gewichtsprozent, vorzugsweise 0,1 bis 1,0 Gewichtsprozent, in dem erfindungsgemäßen Mittel enthalten, wobei als Salz der Salicylsäure vor allem ein Alkalisalicylat oder das Ammoniumsalicylat, insbesondere das Natriumsalicylat, geeignet ist.

Als physiologisch unbedenkliche aliphatische organische Säure der Komponente (C) kommen vorzugsweise von Amino-oder Halogensubstituenten freie aliphatische organische Säuren mit 2 bis 6 Kohlenstoffatomen, wie zum Beispiel Zitronensäure, Weinsäure, Milchsäure, Adipinsäure, Maleinsäure, Glyoxylsäure, Gluconsäure und Äpfelsäure in Betracht, wobei Zitronensäure bevorzugt ist, während als anorganische Säure der Komponente (C) vorzugsweise Phosphorsäure verwendet wird.

Die Komponente (C) ist in den neuen Mitteln in einer Menge von etwa 0,1 bis 1,5 Gewichtsprozent, vorzugsweise 0,25 bis 0,5 Gewichtsprozent, enthalten.

Mit der Komponente (C) wird der pH-Wert des Haar-und Körperreinigungsmittels eingestellt, welcher bei 2,0 bis 7,0, vorzugsweise 4,8 bis 5,8, liegt.

Das erfindungsgemäße Haar-und Körperreinigungsmittel liegt vorzugsweise in Form einer wäßrigen Lösung oder Emulsion vor und enthält neben der Kombination aus (A), (B) und (C) mindestens ein anionisches, kationisches, nicht-ionisches oder amphoteres Tensid in einer Menge von etwa 3 bis 50 Gewichtsprozent, vorzugsweise 10 bis 25 Gewichtsprozent.

Unter den für das neue Haar-und Körperreinigungsmittel geeigenten Tensiden seien beispielsweise die folgenden genannt:

a) Die anionischen oberflächenaktiven Agenzien, wie beispielsweise die Alkali-, Erdalkali-, Ammonium-oder Alkanolaminsalze von Alkansulfonaten, Alkylsulfaten und Alkylethersulfaten, die $C_{12}$ bis $C_{18}$-Alkyl-und insbesondere $C_{12}$ bis $C_{14}$-Alkyl-Sulfatnatriumsalze oder -Triethanolaminsalze, die Natrium-oder Triethanolaminsalze von Lauryl-oder Tetradecylethersulfaten, das Dinatriumsalz des Sulfosuccinhalbesters von Alkanolamiden, die Seifen und die Polyethercarbonsäuren;

b) die nichtionischen oberflächenaktiven Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl und Stearylalkohol, allein oder im Gemisch; die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül; Fettsäurealkanolamide sowie oxethylierte Sorbitanfettsäureester;

c) die kationischen oberflächenaktiven Agenzien, wie beispielsweise das Dilauryldimethylammoniumchlorid, die Chloride oder Bromide von Alkyldimethylbenzylammonium, die Alkyltrimethylammoniumsalze, beispielsweise Cetyltrimethylammoniumchlorid oder -bromid, Tetradecyltrimethylammoniumchlorid oder -bromid, die Alkyldimethylhydroxyethylammoniumchloride oder -bromide, die Dialkyldimethylammoniumchloride oder -bromide, Alkylpyridiniumsalze, beispielsweise Lauryl-oder Cetylpyridiniumchlorid, die Alkylamidethyltrimethylammoniumethersulfate, Imidazolinderivate, Verbindungen mit kationischem Charakter wie Aminoxide, beispielsweise Alkyldimethylaminoxide oder Alkylaminoethyldimethylaminoxide;

d) die amphoteren oder zwitterionischen oberflächenaktiven Agenzien wie beispielsweise die Carboxylderivate von Imidazol, die N-Alkylbetaine, die N-Alkylaminobetaine, die N-Alkylsulfobetaine, die N-Alkylaminopropionate, die Alkyldimethylammoniumacetate, die $C_{12}$ bis $C_{18}$-Alkyldimethylcarboxymethylammoniumsalze sowie die Fettsäurealkylamidobetaine, beispielsweise Dimethylcarboxymethylenpropylenamido-stearat-betain.

Selbstverständlich kann das erfindungsgemäße Mittel neben den genannten Bestandteilen auch übliche kosmetische Zusätze, beispielsweise Parfümöle in einer Menge von etwa 0,5 bis 5,0 Gewichtsprozent, Trübungsmittel, wie zum Beispiel Ethylenglykoldistearat, in einer Menge von etwa 0,5 bis 5,0 Gewichtsprozent, Perlglanzmittel, wie zum Beispiel ein Gemisch aus Fettsäuremonoalkylolamid und Ethylenglykoldistearat, in einer Menge von etwa 1,0 bis 10,0 Gewichtsprozent, Verdickungsmittel, wie beispielsweise Kokosfettsäurediethanolamid, in einer Menge von etwa 0,5 bis 10,0 Gewichtsprozent, Verdünnungsmittel, wie zum Beispiel 1,2-Propylenglykol oder ethoxyliertes Sorbitanmonolaurat, in einer Menge von etwa 0,5 bis 5,0 Gewichtsprozent, Puffersubstanzen, wie beispielsweise Natriumcitrat oder Natriumphosphat, in einer Menge von etwa 0,1 bis 1,0 Gewichtsprozent, Lösungsvermittler, wie zum Beispiel ethoxyliertes, gegebenen falls hydriertes Rizinusöl, in einer Menge von etwa 0,1 bis 1,0 Gewichtsprozent, sowie Farbstoffe, wie zum Beispiel Fluorescein-Natriumsalz, in einer Menge von etwa 0,1 bis 1,0 Gewichtsprozent, weiterhin haar-und hautpflegende Zusätze, wie zum Beispiel Fettsäureester, Fettalkohole, Fettsäureglyceride, ethoxylierte propoxylierte gesättigte Fettalkohole, Cellulosederivate, kationische Cellulosederivate, Chitosan, kationische Chitinderivate, Lanolinderivate, Cholesterin und Pantothensäure in einer Menge von etwa 0,1 bis 10 Gewichtsprozent, außerdem physiologisch verträgliche anorganische Salze, wie zum Beispiel Natriumchlorid, sowie ferner Feuchthaltemittel, Lichtschutzmittel, Antioxidantien, Komplexbildner und Antischuppenwirkstoffe enthalten.

Weitere übliche, für derartige Mittel bekannte Bestandteile, welche in dem neuen Mittel enthalten sein können, sind beispielsweise bei H. Janistyn, "Handbuch der Kosmetika und Riechstoffe", 3. Band, (1973), Seiten 228 - 284 und 442 - 462, K. Schrader, "Grundlagen und Rezepturen der Kosmetika", (1979), Seiten 375 - 401 und 445 - 455 sowie G. A. Nowak, "Die kosmetischen Präparate", (1984), Seiten 452 - 512, beschrieben.

Die nachfolgenden Beispiele sollen den Gegenstand der Erfindung näher erläutern, ohne ihn darauf zu beschränken.

# HAAR-UND KÖRPERREINIGUNGSMITTEL

**Beispiel 1:** Haarreinigungsmittel

```
 0,15 g  Natriumformiat
 0,20 g  Salicylsäure
16,00 g  Laurylalkohol-diglykolethersulfat, Natriumsalz
         (70prozentiges wäßriges Gel)
 0,25 g  kationisches Cellulosederivat (z. B. Polymer
         JR®-400 der Firma Union Carbide Corp.)
 0,30 g  Parfümöl
 3,80 g  Natriumchlorid
79,30 g  Wasser, vollentsalzt
100,00 g
```

Das obige Haarreinigungsmittel, dessen pH-Wert mit Zitronensäure auf 5,0 bis 5,8 eingestellt wird, ist sehr gut konserviert und hervorragend verträglich.

**Beispiel 2:** Haarreinigungsmittel

```
 0,15 g  Natriumformiat
 0,25 g  Natriumsalicylat
16,00 g  Laurylalkohol-diglykolethersulfat, Natriumsalz
         (70prozentiges wäßriges Gel)
 0,25 g  kationisches Cellulosederivat (z. B. Polymer
         JR®-400 der Firma Union Carbide Corp.)
 0,30 g  Parfümöl
 3,80 g  Natriumchlorid
79,25 g  Wasser, vollentsalzt
100,00 g
```

Das obige Haarreinigungsmittel, dessen pH-Wert mit Phosphorsäure auf 5,0 bis 5,8 eingestellt wird, ist sehr gut konserviert und gut verträglich.

**Beispiel 3:** Mildes Körperreinigungsmittel

```
  0,15 g   Natriumformiat
  0,20 g   Salicylsäure
 20,00 g   Laurylalkohol-diglykolethersulfat, Natriumsalz
           (70prozentiges wäßriges Gel)

  5,00 g   Fettsäureamidoalkylbetain
  2,00 g   Kokosfettsäurediethanolamid
  0,50 g   Parfümöl
  4,00 g   Natriumchlorid
 68,15 g   Wasser, vollentsalzt

100,00 g
```

Das obige Körperreinigungsmittel, dessen pH-Wert mit Zitronensäure auf 5,0 bis 5,8 eingestellt wird, ist sehr gut konserviert und ausgezeichnet verträglich.

Alle Prozentangaben dieser Anmeldung stellen, soweit nicht anders angegeben, Gewichtsprozent dar.

## Ansprüche

1. Wasserhaltiges Haar-und Körperreinigungsmittel mit einem Gehalt an 3 bis 50 Gewichtsprozent eines Tensids, dadurch gekennzeichnet, daß es eine Kombination aus

(A) einem physiologisch verträglichen Salz der Ameisensäure,

(B) Salicylsäure oder deren physiologisch verträglichem Salz und

(C) einer physiologisch unbedenklichen anorganischen oder aliphatischen organischen Säure enthält.

2. Mittel nach Anspruch 1, dadurch gekennzeichnet, daß die Komponente (A) das Natriumformiat ist.

3. Mittel nach den Ansprüchen 1 und 2, dadurch gekennzeichnet, daß die Komponente (A) in einer Menge von 0,01 bis 2,0 Gewichtsprozent, vorzugsweise 0,1 bis 1,0 Gewichtsprozent, enthalten ist.

4. Mittel nach den Ansprüchen 1 bis 3, dadurch gekennzeichnet, daß die Komponente (B) in einer Menge von 0,05 bis 2 Gewichtsprozent, vorzugsweise 0,1 bis 1,0 Gewichtsprozent, enthalten ist.

5. Mittel nach den Ansprüchen 1 bis 4, dadurch gekennzeichnet, daß es einen pH-Wert von 2,0 bis 7,0, vorzugsweise von 4,8 bis 5,8, aufweist.

6. Mittel nach den Ansprüchen 1 bis 5, dadurch gekennzeichnet, daß die Komponente (C) Zitronensäure oder Phosphorsäure ist.

7. Mittel nach den Ansprüchen 1 bis 6, dadurch gekennzeichnet, daß die Komponente (C) in einer Menge von 0,1 bis 1,5 Gewichtsprozent, vorzugsweise 0,25 bis 0,5 Gewichtsprozent, enthalten ist.

8. Mittel nach den Ansprüchen 1 bis 7, dadurch gekennzeichnet, daß das Tensid ein anionisches Tensid ist.

9. Verwendung einer Kombination aus

(A) einem physiologisch verträglichen Salz der Ameisensäure,

(B) Salicylsäure oder deren physiologisch verträglichem Salz und

(C) einer physiologisch unbedenklichen anorganischen oder aliphatischen organischen Säure zur Konservierung von Haar-und Körperreinigungsmitteln.

# EUROPÄISCHER RECHERCHENBERICHT

Nummer der Anmeldung

EP 87 11 7726

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| A | ENCYCLOPEDIA OF CHEMICAL TECHNOLOGY, Band 11, 3. Ausgabe, 1979, John Wiley & Sons, New York, US; * Seite 256 * --- | 1-9 | A 61 K 7/00 |
| A | ENCYCLOPEDIA OF CHEMICAL TECHNOLOGY, Band 20, 3. Ausgabe, 1979, John Wiley & Sons, New York, US; * Seite 510, Absatz 2 * ----- | 1-9 | |

RECHERCHIERTE SACHGEBIETE (Int. Cl.3)

A 61 K 7/00
C 11 D 3/00

Der vorliegende Recherchenbericht wurde für alle Patentansprüche erstellt

| Recherchenort | Abschlußdatum der Recherche | Prüfer |
|---|---|---|
| BERLIN | 29-02-1988 | AVEDIKIAN P.F. |

EPO FORM 1503 03.82 (P0403)